# EUROPEAN PATENT APPLICATION

(11) **EP 4 067 506 A1**
(43) Date of publication of application: **05.10.2022**
(21) Application number: 20893105.5
(22) Date of filing: 10.11.2020
(51) Int. Cl.: C12Q 1/6886, G01N 33/574

(54) **BIOMARKER FOR PREDICTING THERAPEUTIC RESPONSIVENESS TO IMMUNE CELL THERAPEUTIC AGENT**

(30) Priority: 29.11.2019 KR 20190156436
(71) Applicant: Sungkwang Medical Foundation, Seoul 06135 (KR)
(72) Inventor: HWANG, So Hyun, Seoul 05823 (KR); LIM, Jae Joon, Suwon-si, Gyeonggi-do 16534 (KR); CHA, Kwang Yul, Seongnam-si Gyeonggi-do 13488 (KR); AN, Hee Jung, Seoul 06098 (KR); KANG, Hae Youn, Seongnam-si, Gyeonggi-do 13496 (KR); CHO, Kyung Gi, Yongin-si Gyeonggi-do 16822 (KR); HEO, Jin Hyung, Gwangju-si, Gyeonggi-do 12787 (KR); JOUNG, Je-Gun, Seoul 04354 (KR); KIM, Se Wha, Seongnam-si, Gyeonggi-do 13489 (KR); KWON, Ah Young, Yongin-si, Gyeonggi-do 16846 (KR)
(74) Representative: Weickmann, Hans
(86) International application number: PCT/KR2020/015670
(87) International publication number: WO 2021/107452

(57) **Abstract**

The disclosure relates to biomarkers for predicting a therapeutic response of a patient to an immune cell therapy. Since gene signatures and markers according to an aspect have an excellent predictive capacity for predicting a patient's therapeutic response to an immune cell therapy, a patient group predicted to exhibit therapeutic effects may be selected to perform an appropriate treatment, and a patient's suffering and costs may be reduced.

## Description

### TECHNICAL FIELD

The present disclosure relates to a biomarker for predicting a therapeutic response to an immune cell therapy.

### BACKGROUND ART

Cancer immunotherapy is an anticancer therapy having a mechanism of activating inhibited immune cells in the body to kill cancer cells, and is a third-generation anticancer therapy that improves the side effects of chemotherapy which is a first-generation anticancer therapy, and the resistance of targeted therapy which is a second-generation anticancer therapy.

A cancer immunotherapy which shows revolutionary clinical effects in various cancers uses an immune checkpoint inhibitor which induces activation of T cells by suppressing binding of PD-L1, which is expressed in cancer cells or peritumoral cells as an immune evasion strategy, and PD-1 expressed in T cells. Currently, anticancer cellular immunotherapies have been actively developed and clinically studied in more than 10 types of cancer, such as brain tumor, malignant melanoma, gastric cancer, urothelial carcinoma, head and neck cancer, liver cancer, and ovarian cancer, and the development of biomarkers to predict a therapeutic response to an immune cell therapy is desperately needed to prevent waste of medical expenditure and to use cancer immunotherapy in the correct way.

Although glioma has the highest incidence among primary malignant brain tumors, the survival period is only about a year even when standard treatment including all of surgery, anti-cancer therapy, and radiation therapy is given to the patient.

Currently, many studies have been conducted on somatic mutations for predicting the prognosis of gliomas, such as IDH1, ATRX, EGFR, and CIC mutations. A previous study showed the possibility that an immune cell therapy may effectively induce the effect of a longer survival period. Although presence or absence of such mutations may be used as a simple indicator for a prognostic prediction, there are many shortcomings for the same to be used as a prognostic predictor for potential therapeutic targets and therapeutic responses.

Therefore, it is important to select a biomarker gene that can predict potential therapeutic targets and therapeutic responses in connection with an immune cell therapy, and a method of precisely evaluating factors related to clinical response, prognosis, and prognostic prediction of a treatment as the cancer progresses is greatly needed, and various enterprises, pharmaceutical companies, and large hospitals are striving to overcome the limitations. In addition, in order for a cancer immunotherapy to be effective, the development of biomarkers including tumor microenvironment genes is needed.

The present inventors analyzed expression data of brain tumor patients who receiving an immune cell therapy and selected biomarkers related to therapeutic prognosis, thereby solving the above problems.

### DESCRIPTION OF EMBODIMENTS

### TECHNICAL PROBLEM

As aspect is to provide a composition for predicting a patient's therapeutic responsereceiving an immune cell therapy including a formulation capable of measuring expression levels of mRNA or a protein thereof of at least one gene selected from the group consisting of APP, C3, CASP1, CD33, CD3E, CD5, CD86, F13A1, FCGR2A, HLA-DRA, HLA-G, ICAM2, IL10, IL10RA, IL12RB2, IL18, IL1B, IL34, IL7, IL7R, INPP5D, ITGA1, JAM3, LAMP3, LY96, NFKB1, NLRP3, NOTCH1, PDCD1, PECAM1, PSMB8, PTPRC, SAA1, SELPLG, ST6GAL1, STAT4, TICAM2, TLR1, TNFRSF18, TNFSF4, and TREM2.

Another aspect is to provide a kit including the composition for predicting a therapeutic response of a patient receiving an immune cell therapy.

Another aspect is to provide a method of providing information for predicting a patient's therapeutic response including: measuring expression levels of mRNA or a protein thereof of at least one gene selected from the group consisting of APP, C3, CASP1, CD33, CD3E, CD5, CD86, F13A1, FCGR2A, HLA-DRA, HLA-G, ICAM2, IL10, IL10RA, IL12RB2, IL18, IL1B, IL34, IL7, IL7R, INPP5D, ITGA1, JAM3, LAMP3, LY96, NFKB1, NLRP3, NOTCH1, PDCD1, PECAM1, PSMB8, PTPRC, SAA1, SELPLG, ST6GAL1, STAT4, TICAM2, TLR1, TNFRSF18, TNFSF4, and TREM2; and predicting therapeutic response to an immune cell therapy based on the measured expression levels of mRNA or a protein thereof.

### SOLUTION TO PROBLEM

As aspect is to provide a composition for predicting a patient's therapeutic response receiving an immune cell therapy including a formulation capable of measuring expression levels of mRNA or a protein thereof of at least one gene selected from the group consisting of APP, C3, CASP1, CD33, CD3E, CD5, CD86, F13A1, FCGR2A, HLA-DRA, HLA-G, ICAM2, IL10, IL10RA, IL12RB2, IL18, IL1B, IL34, IL7, IL7R, INPP5D, ITGA1, JAM3, LAMP3, LY96, NFKB1, NLRP3, NOTCH1, PDCD1, PECAM1, PSMB8, PTPRC, SAA1, SELPLG, ST6GAL1, STAT4, TICAM2, TLR1, TNFRSF18, TNFSF4, and TREM2.

As used herein, the term "gene" may refer to any nucleic acid sequence or a portion thereof having a functional role in coding or transcribing a protein or in regulating expression of other genes. A gene may be consisted of a whole nucleic acid coding or expressing a functional protein or just a portion of the nucleic aicd. A nucleic acid sequence may include a genetic abnormality in an exon, intron, initiation or termination region, promoter sequence, other regulative sequences or a specific sequence adjacent to a gene.

In an embodiment, the gene may be at least one selected from the group consisting of APP, C3, CASP1, CD33, CD3E, CD5, CD86, F13A1, FCGR2A, HLA-DRA, HLA-G, ICAM2, IL10, IL10RA, IL12RB2, IL18, IL1B, IL34, IL7, IL7R, INPP5D, ITGA1, JAM3, LAMP3, LY96, NFKB1, NLRP3, NOTCH1, PDCD1, PECAM1, PSMB8, PTPRC, SAA1, SELPLG, ST6GAL1, STAT4, TICAM2, TLR1, TNFRSF18, TNFSF4, and TREM2.

In an embodiment, the gene may be at least one selected from the group consisting of CD5, CD86, F13A1, HLA-DRA, ICAM2, IL10, IL10RA, IL12RB2, IL34, IL7, IL7R, ITGA1, PECAM1, TNFRSF18, TNFSF18, and TNFSF4.

In an embodiment, the gene may include TNFSF4, TNFRSF18, IL12RB2, or a combination thereof. Preferably, the gene may include TNFSF4, TNFRSF18, and IL12RB2.

In an embodiment, the gene may be CD5, CD86, F13A1, HLA-DRA, ICAM2, IL10, IL10RA, IL12RB2, IL34, IL7, IL7R, ITGA1, PECAM1, TNFRSF18, TNFSF18, and TNFSF4.

The gene may be involved in at least one function selected from the group consisting of a function of pathogen defense, a function of interleukins, leukocytes, chemokines, regulation, cytokines, T cells, B cells, cytotoxicity, a toll-like receptor (TLR), a TNF superfamily, antigen processing, NK cells, microglias, cell cycle, senescence, adhesion, and the complement system, a transporter function, cell function, a function associated to a degenerative disease, neuroinflammation, an inflammasome pathway, or a macrophage.

In an embodiment, the composition may predict a patient's response to a cancer immunotherapy or predict therapeutic prognosis by including a gene related to the function.

As used herein, the term "cancer immunotherapy" refers to an anticancer therapy that kills cancer cells by activating immune cells in the body, or a medicine that shows a therapeutic effect on cancer by strengthening a patient's immunity. In an embodiment, the cancer immunotherapy may be an immune cell therapy.

As used herein, the term "immune cell therapy" may refer to a cancer immunotherapy that attacks cancer cells by activating T cells by blocking activation of a immune checkpoint protein such as PD-L1 expressed in a tumor cell.

In an embodiment, the immune cell therapy may be NK cell therapy, T cell therapy, CAR-immune cell therapy, DC vaccine, CTL therapy, anti-PD-L1, anti-PD-1, and anti-CTLA-4, for example, NK cell therapy.

In an embodiment, the CAR-immune cell therapy may be chimeric antigen receptor-T (CAR T) or chimeric antigen receptor-NK (CAR-NK) cell therapy.

As used herein, the term "chimeric antigen receptor (CAR)" may refer to an artificial membrane binding protein that induces immune cells in response to an antigen and kills cells exhibiting the antigen by stimulating the immune cells.

As used herein, the term "DC vaccine" may refer to a cell therapy including dendritic cells, and the term "CTL therapy" may refer to a cell therapy including cytotoxic T lymphocytes (CTL).

In an embodiment, the immune cell therapy may inhibit binding between PD-L1 and PD-1. Particularly, an antibody that can bind to PD-L1 or PD-1 may be used in an immune cell therapy, for example, the antibody may be monoclonal antibodies, human antibodies, humanized antibodies, or chimeric antibodies.

In the composition, the patient may be a patient with a cancer or tumor, and the tumor may be malignant or benign.

As used herein, the term "cancer" or "tumor" means a state where there is a problem in a regulative function of a cell's normal division, differentiation, and apoptosis, and the cells abnormally over-proliferate and infiltrate to a surrounding tissue or organ to form a lump, and destroy or deform an existing structure.

In an embodiment, the cancer may be one selected from the group consisting of non-small cell lung cancer, small cell lung cancer, melanoma, hodgkin lymphoma, gastric cancer, urothelial carcinoma, head and neck cancer, liver cancer, colon cancer, prostate cancer, pancreatic cancer, liver cancer, testis cancer, ovarian cancer, endometrial cancer, cervical cancer, bladder cancer, brain cancer, breast cancer, and kidney cancer. For example, the cancer may be non-small cell lung cancer, but is not limited thereto.

In an example, the tumor may be brain tumor.

The term "brain tumor" refers to a tumor that occurs in a brain and the central nervous system and may be used interchangeably with "brain cancer". In an embodiment, the cancer may be glioma, glioblastomas, anaplastic astrocytomas, meningiomas, pituitary tumors, schwannomas, CNS lymphoma, oligodendrogliomas, ependymomas, low-grade astrocytomas, medulloblastomas, astrocytic tumors, pilocytic astrocytoma, diffuse astrocytomas, pleomorphic xanthoastrocytomas, subependymal giant cell astrocytomas, anaplastic oligodendrogliomas, oligoastrocytomas, anaplastic oligoastrocytomas, myxopapillary ependymomas, subependymomas, ependymomas, anaplastic ependymomas, astroblastomas, chordoid gliomas of the third ventricle, gliomatosis cerebris, glangliocytomas, desmoplastic infantile astrocytomas, desmoplastic infantile gangliogliomas, dysembryoplastic neuroepithelial tumors, central neurocytomas, cerebellar liponeurocytomas, paragangliomas, ependymoblastomas, supratentorial primitive neuroectodermal tumors, choroids plexus papilloma, pineocytomas, pineoblastomas, pineal parenchymal tumors of intermediate differentiation, hemangiopericytomas, tumors of the sellar region, craniopharyngioma, capillary hemangioblastoma, or primary CNS lymphoma.

As used herein, the term, "treatment or prevention of brain tumor" refers to a treatment or prevention of brain tumor having anti-tumor or anti-cancer efficacy, or effects of increasing a response rate, delaying the disease progression, increasing a survival rate, etc. For example, the anti-tumor efficacy of the brain tumor treatment may include inhibition and delay of brain tumor growth, regression, and reduction of brain tumor, increasing the period until regrowth of brain tumor after a therapeutic termination, delay of brain tumor progression, and the like, but is not limited thereto. The anti-tumor efficacy of brain tumor may include a prophylactic efficacy as well as a therapeutic efficacy on an existing brain tumor.

As used herein, the term "therapeutic response" may mean responsiveness that indicates whether a particular drug, for example, an anticancer agent shows a therapeutic effect on a patient's cancer.

As used herein, the term "prediction of a cancer patient's therapeutic response" may mean predicting in advance before administration of a therapeutic agent whether the administration will be useful for treating cancer, or may mean predicting a therapeutic response to a therapeutic agent by measuring expression of a gene.

As used herein, the term "prediction" may mean judging in advance a particular result, for example, a therapeutic response by identifying expression of a particular gene.

As used herein, the term "formulation capable of measuring expression of a gene" may refer to a formulation identifying an expression level of the gene by measuring directly, or indirectly the gene in a specimen of a cancer patient or expression of a protein coded by the gene, in order to predict a therapeutic response to a cancer immunotherapy. The "expression level of a gene" may be identified by measuring an mRNA expression level of the gene or an expression level of a protein coded by the gene. An expression level of a gene according to an aspect may be used as a marker, specifically, a cancer patient's therapeutic response to cancer immunotherapy may be judged differently according to the expression level of the gene.

As used herein, the term "measurement of an mRNA expression level" may mean a process of identifying presence or absence, or an expression level of mRNA of the gene in a specimen of the subjet, and may be measuring an amount of mRNA. In an embodiment, the measurement of an mRNA expression level may be performed by at least one technique selected from the group consisting of reverse transcription polymerase chain reaction (RT-PCR), competitive RT-PCR, realtime RT-PCR, RNaseprotection assay (RPA), northern blotting, and DNA chip.

In an embodiment, the formulation capable of measuring expression levels of mRNA may be a primer or a probe that specifically binds to the gene.

As used herein, the term "primer" may mean a short nucleic acid sequence which has a short free 3' hydroxyl group, is capable of forming a base pair with a complementary template, and functions as a starting point for duplicating the template strand. A primer can initiate DNA synthesis in an appropriate buffer solution and temperature, in the presence of reagents for polymerization (i.e., DNA polymerase or reverse transcriptase) and four different nucleoside triphosphates. In addition, a primer is a sense and antisense nucleic acid having a sequence of 7 to 50 nucleotides, and additional characteristics may be incorporated that do not change the basic nature of a primer acting as an initiation point of DNA synthesis. A sequence of a primer may not be exactly the same with the sequence of the template, within the range that the primer is sufficiently complementary to the template to be hybridized with the template. A position of the primer or a primer binding site may mean a target DNA fragment to which a primer hybridizes.

As used herein, the term "probe" may mean a nucleic acid fragment such as an RNA or a DNA several bases to hundreds of bases long, which may be specifically bonded to mRNA, and the probe may be labeled so that a presence or absence of a particular mRNA may be identified. A probe may be prepared in the form of an oligonucleotide probe, single stranded DNA probe, doublestranded DNA probe, RNA probe, etc. Hybridization may be performed by using a probe complementary to a polynucleotide of a gene according to an aspect, to predict or assay the reactivity of allulose. Selection of an appropriate probe and hybridization conditions may be modified based on what is known in the art.

The primer or probe may be chemically synthesized by using a phosphoramidite solid support method or other widely known methods. Such a nucleic acid sequence may be modified by using many methods known in the related art. Non-limiting examples of such modifications include methylation, capping, substitution of a natural nucleotide to one or more of its homologues, and modifications between nucleotides, for example, modifications to un-charged linkers such as methylphosphonate, phosphotriester, phosphoramidate, carbamate, etc., or to charged linkers such as phosphorothioate, phosphorodithioate, etc.

As used herein, the term "measurement of a protein expression level" may mean a process of identifying presence or absence, or an expression level of a protein of the gene in a specimen of a subject, and may be measuring an amount of a protein. In an embodiment, the measurement of a protein expression level is performed by one or more techniques selected from the group consisting of western blot, enzyme linked immunosorbent assay (ELISA), radioimmunoassay (RIA), radioimmunodiffusion, Ouchterlony immunodiffusion, rocket immunoelectrophoresis, immunohistochemical staining, immunoprecipitation assay, complement fixation assay, fluorescence activated cell sorter (FACS), and protein chip.

In an embodiment, the formulation capable of measuring expression levels of a protein may be an antibody that specifically binds to the protein of a gene.

As used herein, the term "antibody" is a known terminology in the related art and may refer to a protein molecule specific for a particular epitope on an antigen. Forms of an antibody according to an aspect are not particularly limited and may include a polyclonal antibody, monoclonal antibody, a part of any that has an antigen-binding property, all immunoglobulin antibodies, and also, special antibodies such as humanized antibodies. An antibody according to an aspect may include a functional fragment of the antibody molecule as well as the complete form of 2 full length light chains and 2 full length heavy chains. A functional fragment of the antibody molecule means at the least a fragment which has an antigen binding function and may be, for example, Fab, F(ab'), F (ab')₂, Fv, and the like.

Another aspect provides a composition for predicting a cancer patient's therapeutic response to cancer immunotherapy including a formulation for measuring an expression level of the gene. In the composition, the gene, formulation for measuring an expression level of a gene, measurement of an mRNA expression level, measurement of a protein expression level, cancer immunotherapy, cancer, and prediction of a cancer patient's therapeutic response are as described above.

Another aspect provides a kit including the composition for predicting therapeutic response of a patient receiving an immune cell therapy.

In the kit, the cancer immunotherapy, immune cell therapy, cancer, tumor, therapeutic response, and predition are as described above.

The kit can detect a marker by identifying an mRNA expression level of the gene or an expression level of the protein. The kit according to an aspect may not only include a primer, a probe, and an antibody that selectively recognizes a marker for measuring an expression level of the gene that increases or decreases according to the therapeutic response to a cancer immunotherapy, but also one or more kinds of other compositions, solutions, or devices appropriate for an analysis method.

In an embodiment, the kit for measuring an mRNA expression level of a gene may be a kit including essential elements for performing RT-PCR. The RT-PCR kit may further include, in addition to specific primers for the gene, test tubes or other appropriate containers, reaction buffer solution, deoxyribose nucleotide triphosphates (dNTPs), enzymes such as a Taq-polymerase and reverse transcriptase, DNase and RNase inhibitors, diethylpyrocarbonate treated water (DEPC-water), and sterile water.

In an embodiment, the kit for measuring an expression level of a protein may include a substrate, appropriate buffer solution, coloring enzyme, or secondary antibody marked by a fluorescent material, and coloring substrate for immunological detection of an antibody. For the substrate, a nitrocellulose film, 96-well plate made of polyvinyl resin, 96-well plate made of polystyrene resin and slide glass made of glass, etc. may be used, for a coloring enzyme, a peroxidase, alkaline phosphatase, etc. may be used, for a fluorescent material, FITC, RITC, etc. may be used, and for a coloring substrate solution, 2,2'-azino-bis(3-ethylbenzothiazoline-6-sulfonic acid) (ABTS), o-phenylenediamine (OPD), or tetramethyl benzidine (TMB) may be used.

Another aspect provides a method of providing information for predicting a patient's therapeutic response including: measuring an expression level of an mRNA or a protein thereof of at least one gene selected from the group consisting of APP, C3, CASP1, CD33, CD3E, CD5, CD86, F13A1, FCGR2A, HLA-DRA, HLA-G, ICAM2, IL10, IL10RA, IL12RB2, IL18, IL1B, IL34, IL7, IL7R, INPP5D, ITGA1, JAM3, LAMP3, LY96, NFKB1, NLRP3, NOTCH1, PDCD1, PECAM1, PSMB8, PTPRC, SAA1, SELPLG, ST6GAL1, STAT4, TICAM2, TLR1, TNFRSF18, TNFSF4, and TREM2; and
predicting a therapeutic response to an immune cell therapy based on the measured expression level of mRNA or a protein thereof.

Another aspect provides a method of predicting prognosis of a patient who was administered an immune cell therapy agent including: measuring an expression level of mRNA or a protein thereof of a gene selected from the group consisting of APP, C3, CASP1, CD33, CD3E, CD5, CD86, F13A1, FCGR2A, HLA-DRA, HLA-G, ICAM2, IL10, IL10RA, IL12RB2, IL18, IL1B, IL34, IL7, IL7R, INPP5D, ITGA1, JAM3, LAMP3, LY96, NFKB1, NLRP3, NOTCH1, PDCD1, PECAM1, PSMB8, PTPRC, SAA1, SELPLG, ST6GAL1, STAT4, TICAM2, TLR1, TNFRSF18, TNFSF4 and TREM2; and
comparing the measured expression level with the expression level of mRNA or a protein thereof of a gene of a normal control group.

In the method, the gene, patient, cancer, tumor, measurement of an mRNA expression level, measurement of a protein expression level, cancer immunotherapy, immune cell therapy, prediction of a cancer patient's therapeutic response are as described above.

As used herein, the term "biological specimen" means a specimen obtained from a subject. The biological specimen may include whole blood, plasma, serum, red blood cells, white blood cells (for example, peripheral blood monocytes), ductal fluid, ascites, pleural efflux, nipple aspirate, lymphatic fluid (for example, disseminated tumor cells of the lymph nodes), bone marrow aspirate, saliva, urine, feces (i.e. excretion), sputum, bronchial lavage fluid, tears, fine needle aspirate (for example, harvested by random mammary gland microneedle aspiration), any other bodily fluid, tissue specimen (for example, tumor tissue), for example, a tumor biopsy specimen (for example, a puncture biopsy specimen) or a lymph node (for example, sentinel lymph node biopsy specimen), for example, a specimen obtained by surgical resection of tumors, and cell extracts thereof. In an embodiment, the biological specimen may be whole blood or a component thereof, for example, plasma, serum, or cell pellets. In an embodiment, a specimen may be obtained by isolating circulating cells of solid tumors from whole blood or a cell fraction thereof by using any method known in the art. In an embodiment, a specimen may be, for example, a formalin-fixed paraffin-embedded (FFPE) tumor tissue specimen from solid tumors. In an embodiment, the specimen may be a tumor lysate or an extract prepared from a freezed tissue obtained from a subject with cancer.

As used herein, the term "obtainment" may mean obtaining a nucleic acid specimen or a polypeptide specimen from a biological specimen. The obtaining of a nucleic acid specimen may be performed by a nucleic acid isolation method known in the art. For example, a target nucleic acid may be obtained by amplifying and purifying the same by using polymerase chain reaction (PCR), ligase chain reaction (LCR), transcription mediated amplification, or real-time nucleic acid sequence based amplification (NASBA). Alternatively, a target nucleic acid may be a crude isolated nucleic acid, and obtained from a crushed biological specimen. The obtainment of a polypeptide specimen may be performed by a method of protein extraction or separation in the art.

The term "control group" may be used interchangeably with "negative control". The control group may not respond to a cancer immunotherapy or have a low resposiveness.

When the expression level is higher than the expression level measured in the control group, a process may be further included deciding that the subject has a responsiveness to an immune cell therapy, or has a higher possibility to show a good prognosis after an administration of an immune cell therapy agent.

In an embodiment, the method may futher include a process deciding whether or not to give the cancer immunotherapy to the cancer patient based on the predicted therapeutic response. In an embodiment, the process of predicting a therapeutic response may be judging that the cancer patient has a higher therapeutic responsiveness to the cancer immunotherapy when mRNA or protein expression level of the gene is meausred to be higher than other cancer patients. In an embodiment, the process of deciding whether or not to give the cancer immunotherapy may be determining that the cancer patient receives a cancer immunotherapy when the therapeutic response of the cancer patient is predicted to be higher.

In an embodiment, the patient may be a patient with a tumor, and the tumor may be benign or malignant. The tumor may be brain tumor. For example, the brain tumor may be a glioma.

The patient may be a mammal. Specifically, the patient may be human, dog, cat, parrot, hamster, mouse, horse, cow, monkey, chimpanzee, etc.

In an embodiment, the patient may have received or be receiving a standard treatment. For example, the method is to set a future treatment plan by predicting a patient's response to an immune cell therapy, when an expression of the gene or a protein is measured from the biological specimen obtained from the patient who received a standard treatment.

In another aspect, a system for predicting a therapeutic response to a cancer immunotherapy based on the gene expression analysis data is provided, including:
an information receiver for collecting the patient's gene analysis data;
a predictor predicting a patient's response to a cancer immunotherapy by using the information input through the information receiver, wherein
the gene is at least one selected from the group consisting of APP, C3, CASP1, CD33, CD3E, CD5, CD86, F13A1, FCGR2A, HLA-DRA, HLA-G, ICAM2, IL10, IL10RA, IL12RB2, IL18, IL1B, IL34, IL7, IL7R, INPP5D, ITGA1, JAM3, LAMP3, LY96, NFKB1, NLRP3, NOTCH1, PDCD1, PECAM1, PSMB8, PTPRC, SAA1, SELPLG, ST6GAL1, STAT4, TICAM2, TLR1, TNFRSF18, TNFSF4, and TREM2.

Another aspect provides a recording medium including the system.

In the system, the gene, patient, cancer, tumor, measurement of an mRNA expression level, measurement of a protein expression level, cancer immunotherapy, immune cell therapy, prediction of a cancer patient's therapeutic response are as described above.

### ADVANTAGEOUS EFFECTS OF DISCLOSURE

A genetic marker according to an aspect has an excellent predictive capacity for predicting a therapeutic response to an immune cell therapy, so that a patient group expected to show a therapeutic effect may be selected to perform an appropriate treatment, and thus a patient's suffering and costs may be reduced.

### BRIEF DESCRIPTION OF DRAWINGS

FIG. 1 is a conceptual diagram showing a process of constructing a prediction model by selecting a gene associated with a group in which a therapeutic effect is exhibited.
FIG. 2 shows a heat map of expression patterns of three gene biomarkers in a random forest model made of TNFSF4, TNFRSF18, and IL12R2B.
FIG. 3 is a graph (left) showing classification of a responder group and a non-responder group by using a predictive value from a random forest model made of TNFSF4, TNFRSF18, and IL12R2B, and a graph (right) predicting results of rates of response to an immune cell therapy in a control group which received standard treatment.
FIG. 4 is a diagram showing expression characteristics of 770 genes according to the functional group of the gene in a responder group and a non-responder group.
FIG. 5 is a diagram showing expression characteristics of 16 predictive genes selected from each functional group in a responder group and a non-responder group.
FIGS 6A and 6B use a random forest model using 16 genes selected from the group consisting of each functional group; FIG. 6A classifies responder groups and non-responder groups, and FIG. 6B predicts responses to an immunotherapy in a patient group that received standard treatment.

### MODE OF DISCLOSURE

Hereinafter, the present disclosure will be described in more detail through examples. However, these examples are intended to illustrate the present disclosure, and the scope of the present disclosure is not limited to these examples.

Example 1. Selection of research subjects and deduction of biomarker candidate group.

### 1.1 Selection of research subjects

In order to select biomarkers of brain tumor, brain tumor patients were selected as research subjects. A total of 20 glioma patients were selected, among them, the experimental group was 12 glioma patients who have received NK cell therapy, and the control group was 8 glioma patients who have received a standard treatment. In the experimental group, 5 were responders, and 7 were non-responders to NK therapy. From the patients, formalin-fixed praraffin-embedded (FFPE) samples of 5 µl (100 ng to 300 ng) of RNA before the treatment were obtained to proceed a gene analysis. The patient samples used in the study have all passed the approval review of the Institutional Review Board (IRB File No. 2012-12-172-077).

Specifically, the 5 µl of extracted RNA was prepared as samples by using a NanoString nCounter Analysis System (NanoString Technologies, Inc.), and the expression was measured. For quality control (QR) in the experiment, Nanostring's nSolver software and NanostringQC Pro package were used, and data normalization was performed by also using nSolver software.

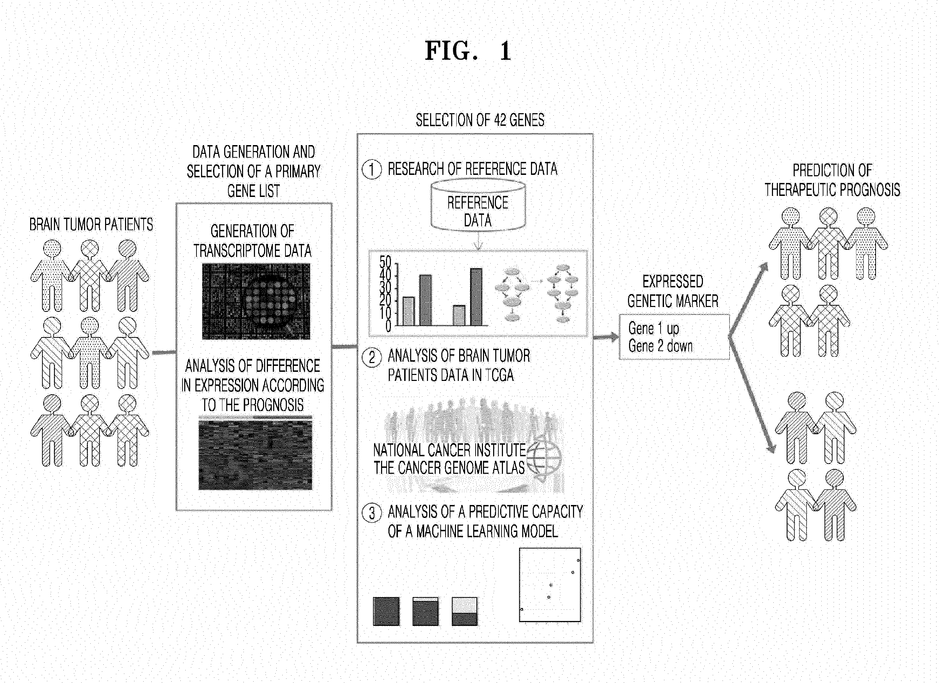

FIG. 1 is a conceptual diagram showing a process of building a prediction model by selecting a gene associated with a group in which a therapeutic effect is exhibited.

Table 1 shows information of the brain tumor patients selected as research subjects.

**[Table 1]**

| | group | # of patients | Overall Survival: Median months(range) | Progression-free Survival: Median months(range) |
|---|---|---|---|---|
| Case | Respond er | 5 | 39(15 ∼ 64) | 24(15 ∼64) |
| | Non-responder | 7 | 11(3 ~ 27) | 5(2 ∼ 12) |
| Control | Standard care | 8 | 15(4 ∼ 51) | NA |

As a result, as shown in Table 1, the responder group compared to the non-responder group among the research subject patients showed a lower recurrence rate, longer period until recorrence, and longer total survival period.

In addition, expression profile data of 770 genes (hereinafter, a biomarker candidate group) showing significant differences in expression between the experimental group and the control group could be deduced.

### 1.2 Selection of biomarkers and method of constructing a model

In order to select expressed gene biomarkers significantly related to a response to NK cell therapy from the biomarker candidate group, transcriptomes and prognosis data of 12 brain tumor patients and data of primary brain tumor patients in The Cancer Genome Atlas (TCGA) were analyzed.

Specifically, two-group t-test and AUC analysis were performed. Next, analyses of overall survival and progressive-free survival was performed. Then, a brain tumor random forest model was constructed by using an R package in random forest. The number of trees in the model was 500, and the rest of the parameters all used default values. For measuring a predictive capacity, a method of leave-one-out cross validation (LOOCV) was used.

Next, statistical analysis was performed on the deduced value. As statistical analysis, violin plot and survival plots were used. The violin plot was drawn using a ggplot2 R package, and the survival plot was analyzed by using a survminer R package. P-value was calculated by using log-rank test. AUC was analyzed by using a ROCR package.

Table 2 shows 41 genes selected for analysis of gene expression data.

**[Table 2]**

| Gene symbol | Full name | Function |
|---|---|---|
| APP | amyloid beta precursor protein | Innate immune response |
| C3 | complement C3 | Innate immune response, |
| CASP1 | caspase 1 | Innate immune response |
| CD33 | CD33 molecule | Myeloid cell surface antigen |
| CD3E | CD3e molecule | Adaptive immune response, T-cell anergy, T-cell differentiation, T-cell proliferation, T cell surface glycoprotein |
| CD5 | CD5 molecule | Adaptive immune response, Regulators of Th1 and Th2 development, T cell surface glycoprotein |
| CD86 | CD86 molecule | Adaptive immune response, Defense response to virus, Regulators of T-cell activation, Th1 & Th2 differentiation, T-cell differentiation, Known as B7-2 |
| F13A1 | coagulation factor XIII A chain | Basic cell function |
| FCGR2A | Fc fragment of IgG receptor IIa | Receptors involved in phagocytosis |
| HLA-DRA | major histocompatibility complex, class II, DR alpha | Adaptive immune response, Antigen processing and presentation |
| HLA-G | major histocompatibility complex, class I, G | Regulation of immune response |
| ICAM2 | major histocompatibility complex, class I, G | Adhesion, CD molecules, Regulation of immune response |
| IL10 | interleukin 10 | Immunosuppression, Interleukins |
| IL10RA | interleukin 10 receptor subunit alpha | CD molecules, Cytokines and receptors |
| IL12RB2 | interleukin 12 receptor subunit beta 2 | Adaptive immune response, Cell Type specific, Cytokines and receptors, Th1 orientation |
| IL18 | interleukin 18 | Interleukins, Th1 orientation |
| IL1B | interleukin 1 beta | Innate immune response, Chemokines and receptors, Cytokines and receptors, Defense response to virus, Interleukins, Regulation of inflammatory response |
| IL34 | interleukin 34 | Innate immune response, Interleukins |
| IL7 | interleukin 7 | Adaptive immune response, Humoral immune response, Interleukins |
| IL7R | interleukin 7 receptor | Adaptive immune response, CD molecules, Cytokines and receptors |
| INPP5D | inositol polyphosphate-5-phosphatase D | Negative regulation of immune response |
| ITGA1 | integrin subunit alpha 1 | Adhesion, CD molecules, T-cell anergy |
| JAM3 | junctional adhesion molecule 3 | Adaptive immune response |
| LAMP3 | lysosomal associated membrane protein 3 | Basic cell functions, Cell Type specific, CD molecules |
| LY96 | lymphocyte antigen 96 | Innate immune response |
| NFKB1 | nuclear factor kappa B subunit 1 | Innate immune response |
| NLRP3 | NLR family pyrin domain containing 3 | Innate immune response |
| NOTCH1 | notch receptor 1 | Transcriptional regulators |
| PDCD1 | programmed cell death 1 | Adaptive immune response, Cell Type specific, CD molecules, Humoral immune response, Negative regulation of immune response |
| PECAM1 | platelet and endothelial cell adhesion molecule 1 | CD molecules, Receptors involved in phagocytosis |
| PSMB8 | proteasome 20S subunit beta 8 | Chemokines and receptors |
| PTPRC | protein tyrosine phosphatase receptor type C | B-cell proliferation, CD molecules, T-cell differentiation |
| SAA1 | serum amyloid A1 | Innate immune response |
| SELPLG | selectin P ligand | CD molecules |
| ST6GAL1 | ST6 beta-galactoside alpha-2,6-sialyltransferase 1 | Humoral immune response |
| STAT4 | signal transducer and activator of transcription 4 | Adaptive immune response, Cytokines and receptors, Cell Type specific, Transcription factors, Transcriptional regulators, Th1 orientation |
| TICAM2 | toll like receptor adaptor molecule 2 | Innate immune response |
| TLR1 | toll like receptor 1 | CD molecules, Innate immune response, Microglial cell activation, Toll-like receptor |
| TNFRSF18 | TNF receptor superfamily member 18 | TNF superfamily members and their receptors, known as GITR |
| TNFSF4 | TNF receptor superfamily member 4 | CD molecules, Chemokines and receptors, TNF superfamily members and their receptors, known as OX40 |
| TRME2 | triggering receptor expressed on myeloid cells 2 | Humoral immune response |

As a result, as shown in Table 2, 41 genetic markers related to a response to NK cell therapy or therapeutic prognosis were selected.

### 1.2 Statistical analysis

A statistical analysis was performed on the 41 genes of Table 2 in the same manner as in Example 1.3.

Table 3 shows AUC, t-test p-value, overall survival p-value, and disease-free survival p-value in the statistical analysis results.

**[Table 3]**

| Gene Symbol | AUC(>=0.9) | t-test p-value(<0.05) | Overall survival p-value(<0.05) | Disease-free survival p-value(<0.05) |
|---|---|---|---|---|
| APP | 0.91 | 0.019 | 0.004 | 0.006 |
| C3 | 0.97 | 0.031 | 0.010 | 0.006 |
| CASP1 | 0.91 | 0.039 | 0.004 | 0.002 |
| CD33 | 0.63 | 0.508 | 0.255 | 0.569 |
| CD3E | 0.94 | 0.300 | 0.014 | 0.003 |
| CD5 | 0.97 | 0.223 | 0.014 | 0.003 |
| CD86 | 1.00 | 0.026 | 0.021 | 0.011 |
| F13A1 | 1.00 | 0.022 | 0.014 | 0.003 |
| FCGR2A | 0.94 | 0.098 | 0.014 | 0.003 |
| HLA-DRA | 1.00 | 0.111 | 0.014 | 0.003 |
| HLA-G | 0.97 | 0.010 | 0.004 | 0.002 |
| ICAM2 | 0.97 | 0.046 | 0.021 | 0.011 |
| IL10 | 1.00 | 0.076 | 0.010 | 0.006 |
| IL10RA | 0.97 | 0.123 | 0.010 | 0.006 |
| IL12RB2 | 0.86 | 0.062 | 0.114 | 0.016 |
| IL18 | 0.91 | 0.179 | 0.005 | 0.023 |
| IL1B | 0.86 | 0.111 | 0.016 | 0.056 |
| IL34 | 1.00 | 0.004 | 0.014 | 0.003 |
| IL7 | 1.00 | 0.016 | 0.014 | 0.003 |
| IL7R | 0.94 | 0.218 | 0.014 | 0.003 |
| INPP5D | 0.86 | 0.061 | 0.076 | 0.128 |
| ITGA1 | 0.94 | 0.021 | 0.014 | 0.003 |
| JAM3 | 0.77 | 0.094 | 0.076 | 0.200 |
| LAMP3 | 0.97 | 0.112 | 0.014 | 0.003 |
| LY96 | 0.83 | 0.086 | 0.029 | 0.053 |
| NFKB1 | 0.94 | 0.010 | 0.076 | 0.128 |
| NLRP3 | 0.86 | 0.081 | 0.005 | 0.023 |
| NOTCH1 | 0.97 | 0.008 | 0.004 | 0.002 |
| PDCD1 | 0.97 | 0.181 | 0.014 | 0.003 |
| PECAM1 | 1.00 | 0.016 | 0.014 | 0.003 |
| PSMB8 | 0.91 | 0.136 | 0.004 | 0.002 |
| TPRC | 0.97 | 0.115 | 0.014 | 0.003 |
| SAA1 | 0.74 | 0.258 | 0.510 | 0.233 |
| SELPLG | 0.83 | 0.077 | 0.035 | 0.088 |
| ST6GAL 1 | 0.83 | 0.083 | 0.035 | 0.088 |
| STAT4 | 0.94 | 0.032 | 0.004 | 0.006 |
| TICAM2 | 0.97 | 0.020 | 0.010 | 0.006 |
| TLR1 | 0.86 | 0.101 | 0.035 | 0.088 |
| TNFRSF 18 | 0.97 | 0.060 | 0.001 | 0.000 |
| TNFSF4 | 0.94 | 0.001 | 0.010 | 0.006 |
| TREM2 | 0.69 | 0.196 | 0.813 | 0.975 |

### Example 2. Construction of random forest model using each gene list and identification of predictive capacity

### 2.1 Construction of random forest model based on molecular biological relationship and identification of predictive capacity

In order to select a gene signature that best predicts a therapeutic response of a patient receiving an immune cell therapy, expression data of 41 genes of Example 1 was classified to 7 types according to the biological relationship, and each random forest model was contructed and the model with the best predictive capacity was identified. Names of 7 models were inflammasome pathway, Inflammation, TNF superfamily, Adhesion signaling, Cytokine, Degenerative disease association factor, and sum.

Table 4 shows a predictive capacity of random forest models constructed by each gene list.

**[Table 4]**

| Gene list name | Gene list | Sensitivity | Specificity | Accuracy |
|---|---|---|---|---|
| inflammasome pathway | NFKB1, NLRP3, CASP1, IL1B, IL18, TLR1, TICAM2, NOTCH1, LY96 | 0.8 | 0.86 | 0.83 |
| neuroinflammation | NFKB1, TLR1, NOTCH1, LY96, IL1B, IL18 | 0.8 | 0.86 | 0.83 |
| TNF superfamily | TNFSF4, TNFRSF18, IL12RB2 | 1.0 | 1.0 | 1.0 |
| Adhesion signaling | ICAM2, PECAM1, JAM3, SELPLG, ST6GAL1 | 0.6 | 0.71 | 0.67 |
| Cytokine | IL10, IL7, IL7R, IL12RB2, IL10RA | 0.8 | 1.0 | 0.92 |
| Degenerative disease association factor | APP, TREM2, CD33, INPP5D, SAA1, IL-1B | 0.6 | 0.71 | 0.67 |
| sum | APP, CASP1, CD33, ICAM2, IL10, IL10RA, IL12RB2, IL18, IL1B, IL7, IL7R, INPP5D, JAM3, LY96, NFKB1, NLRP3, NOTCH1, PECAM1, SAA1, SELPLG, ST6GAL1, TICAM2, TLR1, TNFRSF18, TNFSF4, TREM2 | 0.8 | 1.0 | 0.92 |

As a result, as shown in Table 4, when predicted with a combination of TNFSF4, TNFRSF18 and IL12RB2 genes, the best predictive capacity with sensitivity of 1.0, and the specificity of 1.0 was shown. However, the selected genes were related to NK cell therapy, and considering the point that related genes affect each other, not only these 3 genes but all 41 genes may have important roles in prediction of a therapeutic response.

### 2.2 Verification of predictive capacity of a gene list selected by random forest model

In order to verify a predictive capacity of the gene list selected by a random forest model, a heat map was drawn by using a heat map package with TNFSF4, TNFRSF18 and IL12RB2 which showed the highest predictive capacity in Example 2.1.

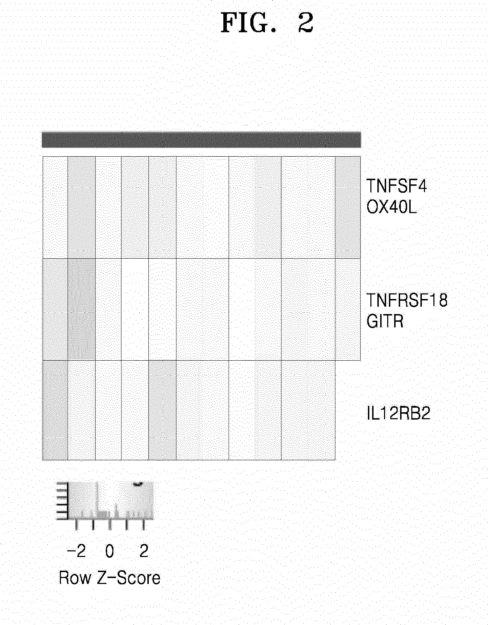

FIG. 2 shows a heat map of expression patterns of three gene biomarkers in a random forest model made of TNFSF4, TNFRSF18, and IL12R2B.

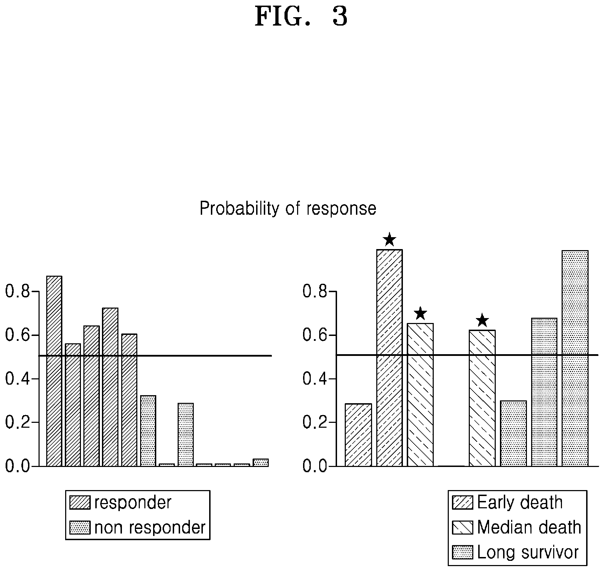

FIG. 3 is a graph (left) showing classification of a responder group and a non-responder group by using a predictive value from a random forest model made of TNFSF4, TNFRSF18, and IL12R2B, and a graph (right) predicting results of a response rate to an immune cell therapy in a control group which received a standard treatment.

As a result, as shown in FIG. 2, expression patterns of three gene biomarkers were identified. It was shown that the expression level of three genes was higher than the non-responder group.

Furthermore, as shown in FIG. 3, probabilities of patients who received an immune cell therapy to respond to the same was shown as rod graphs, and a responder group and a non-responder group could be well distinguished by using the random forest model.

In addition, the probability of response to an immune cell therapy in a control group of patients who received a standard treatment without receiving an immune cell therapy was shown as response rate results, and control group patients marked with red stars in FIG. 3 were found to have a higher probability to show a therapeutic response to an immune cell therapy rather than a standard treatment, and to survive longer.

This means that the genetic biomarker selected in the present disclosure may effectively select patients who will respond to an immune cell therapy.

### Example 3. Functional classification of biomarker candidate group and selection of biomarkers

### 3.1. Functional classification of biomarker candidate group and selection of biomarkers

In order to analyze functional expression characteristics of 770 biomarker candidates derived from Example 1.1, expressions of 770 genes according to the functional group was analyzed in the responder group and the non-responder group.

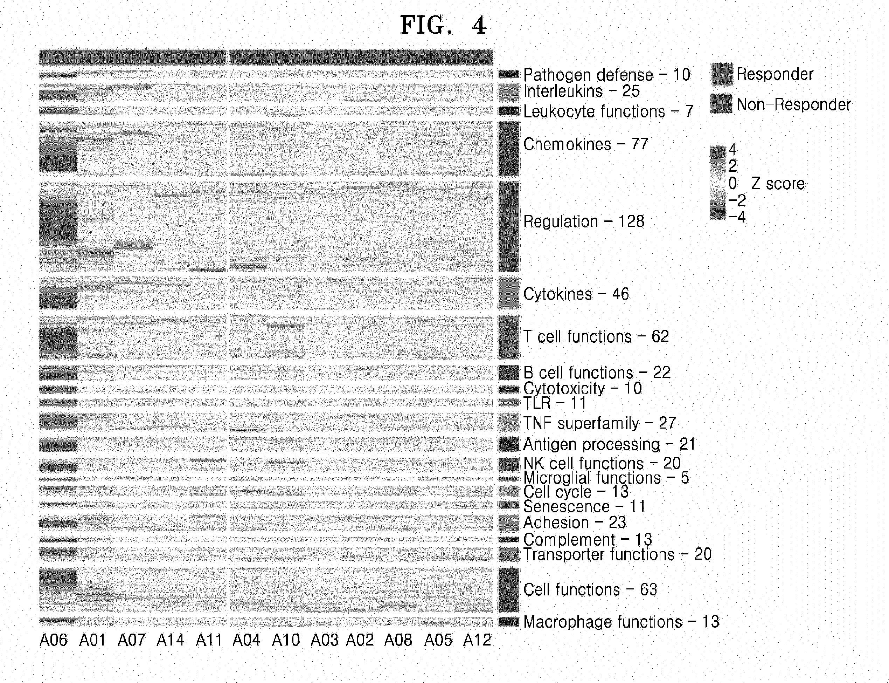

FIG. 4 is a diagram showing expression characteristics of 770 genes according to the functional group of the gene in a responder group and a non-responder group.

As a result, as shown in FIG. 4, expression of immunity-related genes was higher in the responder group.

Next, based on the functional classification of 770 biomarker candidates, genes related to predicting a response to an immune cell therapy were selected. Specifically, genes related to therapeutic prognosis with an AUC of 0.85 or more, an OS p-value of less than 0.05. or a PFS p-value of less than 0.05 were selected. Thereafter, after classifying the selected genes according to the function, a relatively more important gene in prediction in a functional group was decided by calculating importance rank. The importance rank was derived by using a random forest modeling method.

Table 5 is a table selecting 16 genes important in prediction of a response to an immunotherapy from each funtional group.

**[Table 5]**

| | |
|---|---|
| gene | Each function category (importance rank) |
| CD5 | B cells (2nd), T cells (2nd) |
| CD86 | Adaptive immune response (2.5th), B cells (1st), T cells (1st) |
| F13A1 | Among 64 genes (4th) |
| HLA-DRA | Adaptive immune response (1st) |
| ICAM2 | adhesion (1st) |
| IL10 | Interleukin (1.5th) |
| IL10RA | Cytokines (1.5th) |
| IL12RB2 | NK Cells (2nd) |
| IL34 | Interleukin (1.5th) |
| IL7 | Adaptive immune response (2.5th), Interleukin (2.5th) |
| IL7R | Cytokines (1.5th) |
| ITGA1 | NK Cells (1.5th), adhesion (2nd) |
| PECAM1 | Among 64 genes (2nd) |
| TNFRSF18 | TNF (1st) |
| TNFSF18 | TNF (2.5th) |
| TNFSF4 | Chemokines (1st), TNF (2.5th) |

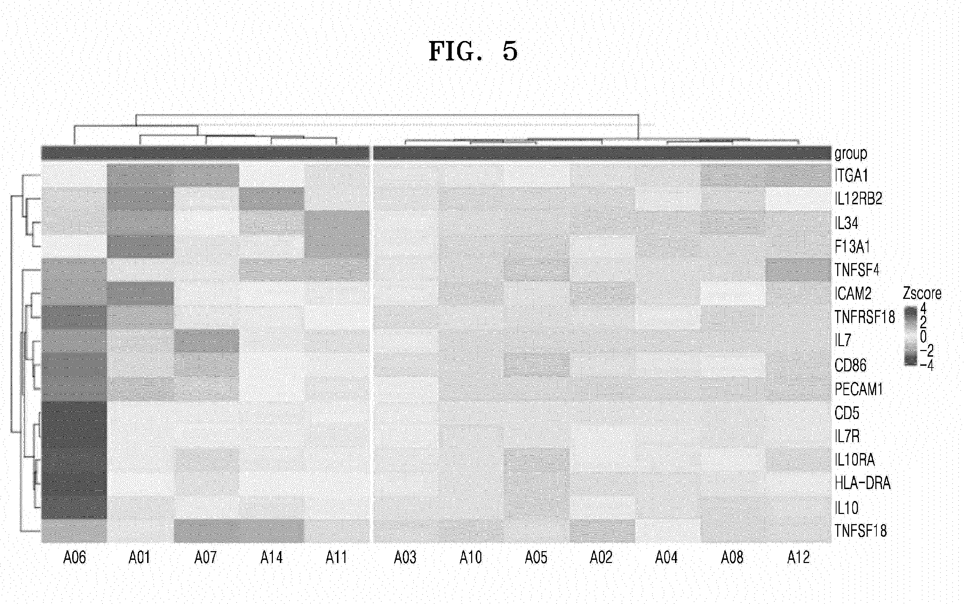

FIG. 5 is a diagram showing expression characteristics of 16 predictive genes selected from the group consisting of each functional group in a responder group and a non-responder group.

As a result, as shown in FIG. 5, in case of the responder group (green), compared to the non-responder group (red), genes selected from the group consisting of each functional group were found to have a higher expression. This means, 16 selected genes may function as markers that effectively determine whether a patient will respond to an immune cell therapy.

### 3.2 Identification of capacity of selected biomarkers to predict response to immunotherapy

In order to identify if the 16 selected genes have capacity to effectively predict a patient's response to an immunotherapy, a random forest model was contructed with the 16 selected genes by using the method in Example 2.

Specifically, a prediction was performed if an immunotherapy would be more effective than a standard treatment for patients who have not received an NK cell immunotherapy. Among the patients who have received a standard treatment, patients who died earlier than an average survival period were marked in orange, and those who lived an average period and died were marked in yellow, and patients who survived longer than the average period were marked in blue.

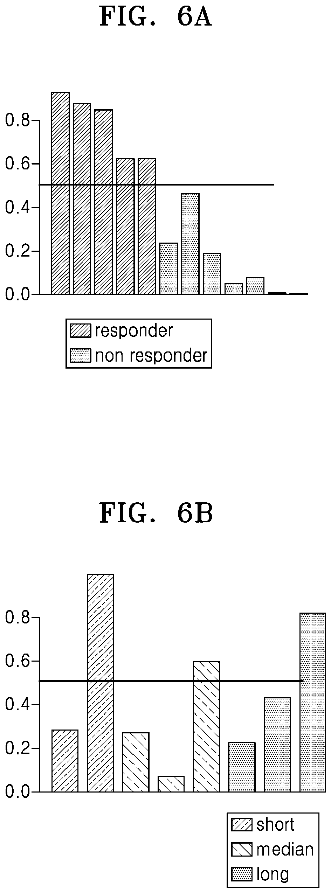

FIGS 6a and 6b use a random forest model using 16 genes selected from the group consisting of each functional group; FIG. 6a classifies a responder group and a non-responder group, and FIG. 6b predicts a response to an immunotherapy in a patient group that received a standard treatment rather than an immunotherapy.

As a result, as shown in FIG. 6, the random forest model constructed with the 16 selected genes was found to be able to distinguish all responders and non-responders. Specifically, when the baseline of the therapeutic response probability was set to 50 %, 3 patients among the 8 patients in the control group were predicted to show a good therapeutic response, and as 2 of the 3 patients had a poor prognosis when a standard treatment was performed, an NK cell immunotherapy rather than a standard therapy was found to have a higher probability to result in a better prognosis. In addition, when a response to an immunotherapy was predicted in patients who had received a standard treatment without an immunotherapy, patients who were expected to respond to an immunotherapy were found to exist regardless of the prognosis after a treatment. These results mean that an immunotherapy rather than a standard treatment may be more useful for patients belonging to a patient group showing early deaths or an average prognosis, and by using the selected gene biomarkers of the present disclosure, the usefulness may be easily judged.

## Claims

1. A composition for predicting a therapeutic response in a patient receiving an immune cell therapy, comprising a formulation capable of measuring expression levels of mRNA or a protein thereof of at least one gene selected from the group consisting of APP, C3, CASP1, CD33, CD3E, CD5, CD86, F13A1, FCGR2A, HLA-DRA, HLA-G, ICAM2, IL10, IL10RA, IL12RB2, IL18, IL1B, IL34, IL7, IL7R, INPP5D, ITGA1, JAM3, LAMP3, LY96, NFKB1, NLRP3, NOTCH1, PDCD1, PECAM1, PSMB8, PTPRC, SAA1, SELPLG, ST6GAL1, STAT4, TICAM2, TLR1, TNFRSF18, TNFSF4, and TREM2.

2. The composition of claim 1, wherein the immune cell therapy is at least one selected from the group consisting of NK cell therapy, T cell therapy, CAR-immune cell therapy, DC vaccine, anti-PD-L1, anti-PD-1, and anti-CTLA-4.

3. The composition of claim 1, wherein the patient is a patient with a tumor.

4. The composition of claim 3, wherein the tumor is a brain tumor.

5. The composition of claim 4, wherein the brain tumor is at least one selected from the group consisting of glioma, glioblastomas, anaplastic astrocytomas, meningiomas, pituitary tumors, schwannomas, CNS lymphoma, oligodendrogliomas, ependymomas, low-grade astrocytomas, medulloblastomas, astrocytic tumors, pilocytic astrocytoma, diffuse astrocytomas, pleomorphic xanthoastrocytomas, subependymal giant cell astrocytomas, anaplastic oligodendrogliomas, oligoastrocytomas, anaplastic oligoastrocytomas, myxopapillary ependymomas, subependymomas, ependymomas, anaplastic ependymomas, astroblastomas, chordoid gliomas of the third ventricle, gliomatosis cerebris, glangliocytomas, desmoplastic infantile astrocytomas, desmoplastic infantile gangliogliomas, dysembryoplastic neuroepithelial tumors, central neurocytomas, cerebellar liponeurocytomas, paragangliomas, ependymoblastomas, supratentorial primitive neuroectodermal tumors, choroids plexus papilloma, pineocytomas, pineoblastomas, pineal parenchymal tumors of intermediate differentiation, hemangiopericytomas, tumors of the sellar region, craniopharyngioma, capillary hemangioblastoma, and primary CNS lymphoma.

6. The composition of claim 1, wherein the formulation capable of measuring expression levels of mRNA is a primer or probe that specifically binds to the gene.

7. The composition of claim 1, wherein the formulation capable of measuring expression levels of a protein is an antibody that specifically binds to the protein of the gene.

8. A kit including the composition of claim 1, for predicting a therapeutic response of a patient receiving an immune cell therapy.

9. A method of providing information for predicting a patient's therapeutic response, comprising:
measuring an expression level of mRNA or a protein thereof of at least one gene selected from the group consisting of APP, C3, CASP1, CD33, CD3E, CD5, CD86, F13A1, FCGR2A, HLA-DRA, HLA-G, ICAM2, IL10, IL10RA, IL12RB2, IL18, IL1B, IL34, IL7, IL7R, INPP5D, ITGA1, JAM3, LAMP3, LY96, NFKB1, NLRP3, NOTCH1, PDCD1, PECAM1, PSMB8, PTPRC, SAA1, SELPLG, ST6GAL1, STAT4, TICAM2, TLR1, TNFRSF18, TNFSF4, and TREM2, from a biological sample obtained from the patient; and
predicting a therapeutic response to an immune cell therapy based on the measured expression level of mRNA or a protein thereof.

10. The method of claim 9, wherein the method futher comprises determining whether or not to give an immune cell therapy to the patient, based on the predicted therapeutic response.

11. The method of claim 9, wherein the predicting of the therapeutic response is judging that the patient has a higher therapeutic response to an immune cell therapy when the measured expression level of mRNA or protein of the gene is increased as compared to that of a patient in a control group.

12. The method of claim 10, wherein the determining of whether or not to give the immune cell therapy to the patient, when the patient's therapeutic response is expected to be high, it is determined that an immune cell therapy is to be given to the patient.

13. The method of claim 9, wherein the patient is a patient with a tumor.

14. The method of claim 13, wherein the tumor is a brain tumor.

15. The method of claim 9, wherein the patient has received or is receiving standard treatment.

16. The method of claim 9, wherein the measurement of the mRNA expression level is performed by at least one technique selected from the group consisting of reverse transcription polymerase chain reaction (RT-PCR), competitive RT-PCR, realtime RT-PCR, RNaseprotection assay (RPA), northern blotting, and DNA chip.

17. The method of claim 9, wherein the measurement of the protein expression level is performed by one or more techniques selected from the group consisting of western blot, enzyme linked immunosorbent assay (ELISA), radioimmunoassay (RIA), radioimmunodiffusion, Ouchterlony immunodiffusion, rocket immunoelectrophoresis, immunohistochemical staining, immunoprecipitation assay, complement fixation assay, fluorescence activated cell sorter (FACS), and protein chip.
